Europäisches Patentamt

European Patent Office

Office européen des brevets

(19)

(11) Numéro de publication: **0 110 749**
**B1**

(12) FASCICULE DE BREVET EUROPEEN

(45) Date de publication du fascicule du brevet:
08.01.86

(51) Int. Cl.⁴: **C 07 C 43/10,** C 07 C 41/03

(21) Numéro de dépôt: **83402091.9**

(22) Date de dépôt: **27.10.83**

(54) Procédé de préparation de produits d'addition d'époxydes et de composés hydroxyles.

(30) Priorité: **17.11.82 FR 8219231**

(43) Date de publication de la demande:
**13.06.84 Bulletin 84/24**

(45) Mention de la délivrance du brevet:
**08.01.86 Bulletin 86/2**

(84) Etats contractants désignés:
**BE DE FR GB IT NL SE**

(56) Documents cités:
**EP - A - 0 013 026**
**DE - A - 2 917 085**

(73) Titulaire: **BP Chimie Société Anonyme, Tour Neptune -
La Défense 1 20, place de Seine, F-92400 Courbevoie
(FR)**

(72) Inventeur: **Falgoux, Daniel, Parc Rigaud 5 allée Canova,
F-13100 Aix en Provence (FR)**
Inventeur: **Simoulin, Danielle, Le Bartavelle Chemin de
Griveton, F-13590 Meyreuil (FR)**
Inventeur: **Pascal-Mousselard, Michel, 4, avenue Henri
Pontier, F-13100 Aix en Provence (FR)**

(74) Mandataire: **Delgrange, Jean Paul, BP Chimie Tour
Neptune Cedex No 20, F-92086 Paris la Défense 1 (FR)**

## Description

La présente invention concerne un procédé de préparation de produits d'addition d'époxydes et de composés hydroxylés, par réaction catalytique en phase liquide homogène.

Il est déjà connu d'effectuer des réactions d'addition d'époxydes et de composés hydroxylés en présence de divers catalyseurs. On sait que l'on obtient par cette réaction un mélange de produits d'addition d'une, de deux ou de plusieurs molécules d'époxyde par molécule de composé hydroxylé. Le produit recherché de préférence étant généralement le produit d'addition comprenant par molécule un seul motif dérivé de l'époxyde, on définit la sélectivité de la réaction d'addition en présence d'un catalyseur, comme étant le rapport en poids de la quantité obtenue de produit d'addition d'une molécule d'époxyde par molécule de composé hydroxylé à la quantité de produit d'addition de deux molécules d'époxyde par molécule de composé hydroxylé, la réaction étant effectuée dans des conditions opératoires déterminées, en présence dudit catalyseur.

Il est déjà connu d'utiliser, comme catalyseurs de cette réaction d'addition, des composés à caractère basique, solubles dans le milieu réactionnel, tels que les hydroxydes des métaux alcalins, ou les alcoolates de ces métaux. Ces catalyseurs sont très actifs, mais ils présentent l'inconvénient de conduire à des réactions peu sélectives.

Il est également connu d'utiliser des catalyseurs à caractère acide, solubles dans les composés hydroxylés. Il est notamment connu d'utiliser des acides forts tels que l'acide sulfurique et les acides sulfoniques ou le trifluorure de bore. Toutefois ces catalyseurs, bien que conduisant à une excellente sélectivité et ayant une activité catalytique élevée, ne peuvent être utilisés dans des réacteurs industriels courants, en raison de leur agressivité vis-à-vis des métaux usuels. De plus, ils donnent lieu à des réactions secondaires indésirables, comportant notamment dans le cas d'utilisation d'oxyde d'éthylène la formation de dioxanne-1-4, substance toxique.

D'autres catalyseurs constitués de sels minéraux neutres, solubles dans les composés hydroxylés, ont également déjà été utilisés, tels que le fluoborate de sodium qui conduit à une haute sélectivité, mais possède une activité catalytique relativement faible. On peut également mentionner des perchlorates, tels que les perchlorates de magnésium, de calcium, de manganèse, de nickel et de zinc qui sont à la fois très actifs et très sélectifs. Ces sels sont peu corrosifs et seraient certainement d'un grand intérêt, si leur usage industriel ne présentait pas des risques importants d'explosion, dus à des décompositions incontrôlables, notamment lorsqu'ils sont concentrés et soumis à une haute température au cours de l'opération de purification par distillation des produits de la réaction d'addition. Les phosphomolybdates et l'acide phosphomolybdique qui sont également des catalyseurs connus, présentent l'avantage d'être très sélectifs, peu corrosifs et sans danger. Malheureusement, ce sont des catalyseurs relativement peu actifs qui doivent être utilisés à fortes concentrations pour être efficaces.

On connaît également des procédés catalytiques de préparation en phase hétérogène de produits d'addition d'époxydes avec des composés hydroxylés, ces procédés utilisant des catalyseurs insolubles dans le milieu réactionnel. De tels procédés sont, en général, mis en œuvre dans des installations très complexes et très différentes de celles utilisées en catalyse homogène.

Parmi les catalyseurs insolubles dans les composés hydroxylés, on peut en particulier citer les résines fluoroalcoyle sulfoniques qui conduisent à des réactions très sélectives, notamment entre l'oxyde d'éthylène et l'eau, le méthanol ou l'éthanol. Malheureusement, ces résines à caractère acide doivent être utilisées à des températures relativement faibles, comprises en pratique entre 50 et 100°C, du fait de leur instabilité thermique. Dans ces conditions, la vitesse de réaction reste relativement faible.

La demanderesse a maintenant trouvé des catalyseurs pour la préparation de produits d'addition d'un époxyde et d'un composé hydroxylé, ces catalyseurs solubles dans le milieu réactionnel ayant à la fois une activité extrêmement élevée et une haute sélectivité, mais ne présentant par ailleurs pas de risques d'explosion et n'étant pas corrosifs vis-à-vis des métaux usuels. Ces catalyseurs peuvent, en outre, être utilisés dans un très large domaine de température, s'étendant de 40 à 250°C et par exemple de 80 à 250°C.

La présente invention a donc pour objet un procédé de préparation de produits d'addition d'époxydes et de composés hydroxylés, caractérisé en ce qu'il comporte la réaction, en phase liquide homogène, à une température comprise entre 40 et 250°C, d'une part, d'un époxyde, choisi parmi les oxydes d'alcoylène et l'épichlorhydrine et, d'autre part, d'un composé hydroxylé choisi parmi les alcools, les phénols et l'eau, ladite réaction étant effectuée en présence d'un catalyseur constitué par un sel de l'acide trifluorométhane sulfonique (ou acide triflique), soluble dans le milieu réactionnel.

Selon l'invention, une grande variété d'oxydes d'alcoylène peut être utilisée; on emploie de préférence l'oxyde d'éthylène, l'oxyde de propylène ou l'oxyde de butylène.

On peut choisir les composés hydroxylés mis en œuvre selon l'invention parmi un grand nombre de composés choisis parmi les alcools, les phénols et l'eau. Les alcools mis en œuvre peuvent être des alcools aliphatiques primaires ou secondaires. On préfère généralement utiliser des alcools aliphatiques primaires, tels que le méthanol, l'éthanol, le propanol et le n-butanol. Toutefois, de très bons résultats peuvent également être obtenus avec des alcools aliphatiques primaires plus lourds comportant jusqu'à 20 atomes de carbone, comme par exemple le n-octanol ou le dodécanol, ou avec les alcools aliphatiques secondaires tels que l'isopropanol ou le butanol secondaire, ou les monoéthers d'alcoylèneglycol alors que l'on sait que les catalyseurs utilisés jusqu'à présent, notamment les catalyseurs basiques, sont relativement peu actifs et peu sélectifs dans les réactions d'addition des époxydes sur ces alcools.

Selon l'invention, le composé hydroxylé est généralement utilisé en large excès pondéral par rapport à l'époxyde, le rapport pondéral de la quantité de composé hydroxylé à celle d'époxyde étant par exemple compris entre 2 et 20.

Comme sel de l'acide triflique (trifluorométhane sulfonate ou triflate), on utilise de préférence le triflate d'aluminium. Toutefois, on peut également utiliser un triflate d'un métal alcalin, tel que par exemple le triflate de lithium, un triflate d'un métal alcalino-terreux, un triflate d'un métal du groupe II de la classification périodique des éléments, tel que le triflate de magnésium de préférence le triflate de zinc, un triflate d'un métal lourd tel que, par exemple, un triflate de cobalt, de nickel, de zirconium, d'étain, ou un triflate de tétraalcoylammonium.

Les triflates utilisés comme catalyseurs selon l'invention peuvent être obtenus facilement selon des procédés de préparation bien connus en eux-mêmes. En particulier, les triflates des métaux cités ci-dessus peuvent être préparés par action de l'acide triflique sur ces métaux ou sur un oxyde, un hydroxyde ou un carbonate desdits métaux. La plupart des triflates possèdent une excellente stabilité thermique et ne se décomposent qu'à température élevée, généralement supérieure à 300° C.

La quantité de triflate mise en œuvre doit être suffisante pour obtenir l'effet catalytique désiré. Dans la pratique, la quantité de triflate utilisée est généralement extrêmement faible, très inférieure aux quantités de catalyseur qui doivent être utilisées pour obtenir les mêmes vitesses de réactions dans les mêmes conditions opératoires, lorsqu'on utilise les catalyseurs antérieurement connus.

La quantité de triflate utilisée peut varier entre 1 et 100 ppm en poids du mélange réactionnel, ces limites étant fonction notamment de la nature des réactifs en présence, de la température de la réaction et des temps de séjour. Dans le cas, par exemple, d'utilisation d'alcools primaires comportant de 1 à 6 atomes de carbone, l'effet catalytique est déjà sensible pour une concentration en catalyseur égale à 1 ppm par rapport au mélange réactionnel; toutefois, des concentrations comprises entre 2 et 50 ppm sont généralement préférées. Dans le cas de réactions faisant intervenir des alcools supérieurs, comportant au moins 7 atomes de carbone, ou d'autres composés organiques hydroxylés comme les phénols et les monoéthers d'alcoylène-glycols, les concentrations en triflate dans le mélange réactionnel doivent généralement être comprises entre 10 et 100 ppm.

La réaction se déroule en phase homogène, à une température comprise de préférence entre 60 et 200° C, et en particulier entre 80 et 150° C, sous une pression suffisante pour que le mélange réactionnel soit maintenu à l'état liquide, par exemple sous une pression comprise entre 2,5 et 4,0 MPa. On a trouvé notamment que les triflates conservent, du fait de leur grande stabilité thermique, toute leur activité et leur sélectivité jusqu'à des températures de l'ordre de 250° C. La réaction peut, en outre, être réalisée dans des appareillages courants tels que, par exemple, des autoclaves en acier munis d'agitateur ou des réacteurs tubulaires, pouvant fonctionner sous pression.

Les exemples qui suivent ont pour but d'illustrer la présente invention.

*Exemple 1 :*

a) *Préparation du catalyseur (triflate d'aluminium)*

Dans un ballon en verre de 250 ml, on introduit sous agitation 0,2 g d'aluminium en poudre et 70 ml d'eau. La suspension obtenue est portée à 80° C, puis on ajoute goutte à goutte 33,3 ml d'une solution aqueuse d'acide triflique 0,57N. Ce mélange est maintenu sous agitation à 80° C pendant 2 h, puis à la température ambiante pendant 48 h. Le triflate d'aluminium obtenu peut être ensuite isolé en éliminant par filtration l'excès d'aluminium et en évaporant l'eau à 100° C sous pression atmosphérique. On récupère ainsi 3,5 g de triflate d'aluminium, de formule $Al(CF_3SO_3)_3$, sous forme d'une poudre blanche.

b) *Préparation du mélange réactionnel à température ambiante*

Dans un récipient en acier de 5 l, muni d'un système d'agitation, on introduit 1620 g de n-butanol et 18 mg du triflate d'aluminium préparé précédemment. Le mélange obtenu est soumis à un balayage à l'azote gazeux, afin d'éliminer l'air présent. On introduit ensuite 180 g d'oxyde d'éthylène et on maintient l'agitation afin d'homogénéiser le mélange. La concentration en triflate d'aluminium dans le mélange réactionnel est égale à 10 ppm.

c) *Fabrication du monobutyléther d'éthylène-glycol*

Le mélange réactionnel obtenu ci-dessus alimente au moyen d'une pompe doseuse un réacteur tubulaire constitué d'un tube en acier inoxydable de diamètre intérieur 4 mm et de 50 m de long. Le réacteur tubulaire est placé dans une enceinte thermique, maintenue à 200° C. La pression à l'intérieur du réacteur est maintenue constante à 3 MPa. On règle l'alimentation du réacteur tubulaire de telle sorte que le temps de séjour moyen du mélange réactionnel dans le réacteur soit égal à 2 h.

Après passage dans le réacteur tubulaire, le mélange est refroidi au travers d'un serpentin réfrigérant; un dispositif d'analyse automatique par chromatographie permet de déterminer la composition du mélange final.

Les résultats sont fournis dans le tableau I. On constate que la transformation de l'oxyde d'éthylène est complète, c'est-à-dire que le taux de conversion de l'oxyde d'éthylène est égal à 1,00. Les quantités de monobutyléthers de mono-, di- et triéthylèneglycol en fin de réaction (exprimées en pourcentage en poids par rapport au milieu réactionnel) sont respectivement égales à 20,3%, 2,6% et 0,1%. Le rapport pondéral de la quantité produite de monobutyléther de monoéthylèneglycol à celle de monobutyléther de diéthylèneglycol ou sélectivité S de la réaction est donc égal à 7,8, ce qui est une valeur relativement élevée.

*Exemple 2 (comparatif):*

A titre de comparaison, un essai a été réalisé dans des conditions opératoires identiques à celles de l'exemple 1, excepté le fait qu'à la place du triflate d'aluminium à la concentration de 10 ppm, on utilise l'acétate de potassium, de formule $CH_3COOK$, à la concentration de 50 ppm.

Les résultats donnés dans le tableau I montrent que, malgré une concentration en catalyseur très supérieure à celle de l'exemple 1, le taux de conversion de l'oxyde d'éthylène est seulement égal à 0,93. De son côté, la sélectivité de la réaction en présence de l'acétate de potassium, égale à 3,8, est très inférieure à celle obtenue avec le triflate d'aluminium.

*Exemples 3, 4 et 5:*

Dans ces exemples, les conditions opératoires sont identiques à celles de l'exemple 1, excepté le temps de séjour moyen du mélange réactionnel dans le réacteur tubulaire qui est de 1 h dans les trois exemples, et la température de l'enceinte dans laquelle est placée le réacteur tubulaire qui est maintenue respectivement à 150 et 120°C dans les exemples 4 et 5.

Les résultats apparaissent dans le tableau I.

*Tableau I*

| Exemple | Catalyseur | | Tempé-rature (°C) | Temps de séjour moyen (h) | Taux de conversion de l'oxyde d'éthylène[1] | S (sélecti-vité)[2] |
| | Nature | Concen-tration (ppm) | | | | |
|---|---|---|---|---|---|---|
| 1 | $Al(CF_3SO_3)_3$ | 10 | 200 | 2 | 1,00 | 7,8 |
| 2 | $CH_3COOK$ | 50 | 200 | 2 | 0,93 | 3,8 |
| 3 | $Al(CF_3SO_3)_3$ | 10 | 200 | 1 | 1,00 | 7,8 |
| 4 | $Al(CF_3SO_3)_3$ | 10 | 150 | 1 | 1,00 | 6,7 |
| 5 | $Al(CF_3SO_3)_3$ | 10 | 120 | 1 | 1,00 | 6,7 |

[1] Taux de conversion de l'oxyde d'éthylène: rapport de la quantité d'oxyde d'éthylène qui a réagi à celle mise en œuvre.

[2] Sélectivié, définie par: $S = \dfrac{\text{Poids de monobutyléther de monoéthylèneglycol produit}}{\text{Poids de monobutyléther de diéthylèneglycol produit}}$

L'analyse de ces résultats montre clairement l'activité catalytique extrêmement élevée du triflate d'aluminium et la haute sélectivité de la réaction conduite en présence de ce catalyseur. On peut en effet constater en particulier que, pour une concentration en catalyseur aussi faible que 10 ppm, le triflate d'aluminium permet une conversion totale de l'oxyde d'éthylène, même à une température relativement basse et pour un temps de séjour relativement court. On peut constater en outre que la sélectivité de la réaction croît avec la température.

*Exemples 6 à 11 (comparatifs):*

A titre de comparaison, des essais ont été réalisés dans des conditions opératoires identiques à celles de l'exemple 1, excepté le fait qu'à la place du triflate d'aluminium à la concentration de 10 ppm on utilise:

— aux exemples 6 et 7, l'acétate de potassium ($CH_3COOK$), à des concentrations respectivement de 100 et 300 ppm;

— aux exemples 8 et 9, le perchlorate de magnésium, de formule $Mg(ClO_4)_2$, à des concentrations respectivement de 100 et 300 ppm;

— aux exemples 10 et 11, le perchlorate de zinc, de formule $Zn(ClO_4)_2$, à des concentrations respectivement de 100 et 300 ppm.

Les résultats des exemples 6 à 11, ainsi que ceux des exemples 1 et 2, sont rassemblés dans le tableau II.

*(Tableau en page suivante)*

L'examen de ce tableau montre que seul le triflate d'aluminium présente, à très faible concentration, à la fois une activité très élevée et une bonne sélectivité. En effet, les exemples 2, 6 et 7 montrent à titre de comparaison que l'activité de l'acétate de potassium, mesurée par le taux de conversion de l'oxyde d'éthylène est relativement satisfaisant à partir de concentrations en catalyseur de 50 ppm, mais que la sélectivité S des réactions est alors très faible. De leur côté, les perchlorates de magnésium (exemples 8 et 9) ou de zinc (exemples 10 et 11) sont très sélectifs, mais leur activité est médiocre.

*Exemples 12 à 21:*

Dans ces exemples, on opère comme à l'exemple 1 excepté que:

— aux exemples 12 et 13, la température de l'enceinte dans laquelle est placé le réacteur tubulaire est fixée respectivement à 150 et 120°C;

— aux exemples 14 et 15, on utilise l'acétate de potassium ($CH_3COOK$) à la concentration de 50 ppm et on fixe la température de l'enceinte respectivement à 150 et 120°C;

*Tableau II*

| Exemple | Catalyseur | | Taux de conversion de l'oxyde d'éthylène | Teneur en $C_4H_9O-[CH_2CH_2O]_nH$ [1] (% en poids) | | | S (sélectivité) |
|---|---|---|---|---|---|---|---|
| | Nature | Concen- tration (ppm) | | n=1 | n=2 | n⩾3 | |
| 1 | $Al(CF_3SO_3)_3$ | 10 | 1,00 | 20,3 | 2,6 | 0,1 | 7,8 |
| 2 | $CH_3COOK$ | 50 | 0,93 | 16,3 | 4,3 | — | 3,8 |
| 6 | » | 100 | 1,00 | 16,6 | 4,6 | — | 3,6 |
| 7 | » | 300 | 1,00 | 15,3 | 4,3 | 1,4 | 3,5 |
| 8 | $Mg(ClO_4)_2$ | 100 | 0,78 | 17,0 | 1,4 | — | 12,1 |
| 9 | » | 300 | 0,98 | 23,4 | 1,8 | — | 13,0 |
| 10 | $Zn(ClO_4)_2$ | 100 | 0,67 | 16,3 | 1,1 | — | 14,8 |
| 11 | » | 300 | 1,00 | 22,5 | 2,5 | 0,1 | 9,0 |

[1] Pourcentage en poids dans le milieu réactionnel à la sortie du réacteur des quantités respectivement produites de monobutyléther de monoéthylèneglycol (n=1), de monobutyléther de diéthylèneglycol (n=2) et de monobutyléther de polyéthylèneglycol (n⩾3).

— aux exemples 16 et 17, on utilise l'acétate de potassium ($CH_3COOK$) à la concentration de 300 ppm et on fixe la température de l'enceinte respectivement à 150 et 120°C;

— aux exemples 18 et 19, on utilise le perchlorate de magnésium ($Mg(ClO_4)_2$) à la concentration de 300 ppm et on fixe la température de l'enceinte respectivement égale à 150 et 120°C;

— aux exemples 20 et 21, on utilise le perchlorate de zinc ($Zn(ClO_4)_2$) à la concentration de 300 ppm et on fixe la température de l'enceinte respectivement à 150 et 120°C.

Les résultats des exemples 12 à 21, ainsi que ceux des exemples 1, 2, 7, 9 et 11 sont rassemblés dans le tableau III.

*Tableau III*

| Exemple | Catalyseur | | Tempé- rature (°C) | Taux de conversion de l'oxyde d'éthylène | S (sélecti- vité) |
|---|---|---|---|---|---|
| | Nature | Concen- tration (ppm) | | | |
| 1 | $Al(CF_3SO_3)_3$ | 10 | 200 | 1,00 | 7,8 |
| 12 | $Al(CF_3SO_3)_3$ | 10 | 150 | 1,00 | 6,7 |
| 13 | $Al(CF_3SO_3)_3$ | 10 | 120 | 1,00 | 6,7 |
| 2 | $CH_3COOK$ | 50 | 200 | 0,93 | 3,8 |
| 14 | $CH_3COOK$ | 50 | 150 | 0,65 | — |
| 15 | $CH_3COOK$ | 50 | 120 | 0,37 | — |
| 7 | $CH_3COOK$ | 300 | 200 | 1,00 | 3,5 |
| 16 | $CH_3COOK$ | 300 | 150 | 0,98 | 4,0 |
| 17 | $CH_3COOK$ | 300 | 120 | 0,89 | — |
| 9 | $Mg(ClO_4)_2$ | 300 | 200 | 0,98 | 12,5 |
| 18 | $Mg(ClO_4)_2$ | 300 | 150 | 0,71 | — |
| 19 | $Mg(ClO_4)_2$ | 300 | 120 | 0,42 | — |
| 11 | $Zn(ClO_4)_2$ | 300 | 200 | 1,00 | 9,1 |
| 20 | $Zn(ClO_4)_2$ | 300 | 150 | 0,96 | 9,1 |
| 21 | $Zn(ClO_4)_2$ | 300 | 120 | 0,86 | — |

L'analyse de ce tableau montre clairement les avantages que représente l'utilisation de triflate d'aluminium comme catalyseur de la synthèse de monobutyléther de monoéthylèneglycol, notamment lorsqu'on fait varier la température entre 120 et 200°C. On constate que le triflate d'aluminium est le seul catalyseur qui, bien qu'utilisé à une concentration extrêmement faible, possède à la fois une forte sélectivité et une très haute activité dans une gamme de température étendue entre 120 et 200°C, cette haute activité étant mise en évidence par le taux de conversion égal à 1,00.

### Exemples 22 et 23:

Dans ces exemples, les conditions opératoires sont identiques à celles de l'exemple 1, excepté le fait que dans la préparation du mélange réactionnel, on remplace 1620 g de n-butanol par le même poids de méthanol, que la température de l'enceinte dans laquelle est placé le réacteur tubulaire est maintenue à 150°C, qu'en outre, à l'exemple 23, on utilise à la place du triflate d'aluminium à la concentration de 10 ppm, l'acétate de potassium à la concentration de 200 ppm.

Le tableau IV montre les résultats de la fabrication de monométhyléther de monoéthylèneglycol.

*Tableau IV*

| Exemple | Catalyseur | | Tempé-rature (°C) | Taux de conversion de l'oxyde d'éthylène | S (sélecti-vité) |
|---|---|---|---|---|---|
| | Nature | Concen-tration (ppm) | | | |
| 22 | $Al(CF_3SO_3)_3$ | 10 | 150 | 1,00 | 20,0 |
| 23 | $CH_3COOK$ | 200 | 150 | 1,00 | 14,3 |

L'analyse de ces résultats montre l'activité catalytique extrêmement élevée du triflate d'aluminium dans la réaction entre le méthanol et l'oxyde d'éthylène, ainsi que la haute sélectivité de la réaction en monométhyléther du monoéthylèneglycol.

### Exemples 24 et 25:

Dans ces exemples, les conditions opératoires sont identiques à celles de l'exemple 1, excepté le fait que, dans la préparation du mélange réactionnel, on utilise 1620 g d'éthanol à la place de 1620 g de butanol, que la température de l'enceinte, dans laquelle est placé le réacteur tubulaire, est maintenue à 150°C, et qu'en outre, à l'exemple 25, on utilise à la place du triflate d'aluminium à la concentration de 10 ppm, l'acétate de potassium à la concentration de 200 ppm.

Le tableau V montre les résultats de la fabrication de monoéthyléther de monoéthylèneglycol.

*Tableau V*

| Exemple | Catalyseur | | Tempé-rature (°C) | Taux de conversion de l'oxyde d'éthylène | S (sélecti-vité) |
|---|---|---|---|---|---|
| | Nature | Concen-tration (ppm) | | | |
| 24 | $Al(CF_3SO_3)_3$ | 10 | 150 | 1,00 | 10,0 |
| 25 | $CH_3COOK$ | 200 | 150 | 1,00 | 6,7 |

L'analyse de ces résultats montre l'activité très élevée du triflate d'aluminium, comparée à celle de l'acétate de potassium, dans la réaction entre l'éthanol et l'oxyde d'éthylène. En outre, le triflate d'aluminium est très supérieur à l'acétate de potassium, en ce qui concerne la sélectivité de la réaction en monoéthyléther de monoéthylèneglycol.

### Exemples 26 à 29:

Dans ces exemples, les conditions opératoires sont identiques à celles de l'exemple 1, excepté le fait que, dans la préparation du mélange réactionnel, on utilise 1620 g de méthanol à la place de 1620 g de butanol et 180 g d'oxyde de propylène à la place de 180 g d'oxyde d'éthylène, qu'aux exemples 27 et 29, la température de l'enceinte dans laquelle est placé le réacteur tubulaire est maintenue à 150°C, au lieu de 200°C, et qu'en outre, aux exemples 28 et 29, on utilise à la place du triflate d'aluminium à la concentration de 10 ppm, l'acétate de potassium à la concentration de 200 ppm.

Le tableau VI montre les résultats de la fabrication de monométhyléther de monopropylèneglycol.

*(Tableau en page suivante)*

Comme le montrent ces résultats, on constate que le triflate d'aluminium présente une activité très supérieure à celle de l'acétate de potassium dans la réaction entre le méthanol et l'oxyde de propylène, tout en maintenant une sélectivité élevée à haute température pour la production de mo-

*Tableau VI*

| Exemple | Catalyseur | | Tempé-rature (°C) | Taux de conversion de l'oxyde | S (sélecti-vité) |
|---------|------------|--|-------------------|-------------------------------|------------------|
| | Nature | Concen-tration (ppm) | | | |
| 26 | $Al(CF_3SO_3)_3$ | 10 | 200 | 1,00 | 42,0 |
| 27 | $Al(CF_3SO_3)_3$ | 10 | 150 | 1,00 | 29,5 |
| 28 | $CH_3COOK$ | 200 | 200 | 1,00 | 40,0 |
| 29 | $CH_3COOK$ | 200 | 150 | 1,00 | 52,5 |

nométhyléther de monopropylèneglycol. On constate, en particulier, que la sélectivité de la réaction en présence de triflate d'aluminium croît d'une façon inattendue avec la température, contrairement à ce que l'on observe pour les réactions en présence d'acétate de potassium.

*Exemple 30:*

a) *Préparation du catalyseur (triflate de zinc)*

Dans un ballon en verre de 250 ml, on introduit sous agitation 0,5 g de zinc en poudre et 70 ml d'eau. La suspension obtenue est portée à 80°C, puis on ajoute goutte à goutte 22,2 ml d'une solution aqueuse d'acide triflique 0,57N. Ce mélange est maintenu sous agitation à 80°C pendant 2 h, puis à la température ambiante pendant 48 h. Le triflate de zinc ainsi obtenu peut être ensuite isolé en éliminant par filtration l'excès de zinc et en évaporant l'eau à 100°C sous pression atmosphérique. On récupère ainsi près de 3 g de triflate de zinc, de formule $Zn(CF_3SO_3)_2$, sous forme d'une poudre blanche.

b) *Préparation du mélange réactionnel à température ambiante*

On opère exactement comme dans l'exemple 1, excepté le fait qu'au lieu d'introduire 18 mg de triflate d'aluminium dans le récipient, on introduit 90 mg du triflate de zinc préparé précédemment. La concentration en triflate de zinc dans le mélange réactionnel est donc égale à 50 ppm.

c) *Fabrication de monobutyléther d'éthylèneglycol*

On opère exactement comme dans l'exemple 1, en alimentant le réacteur tubulaire au moyen du mélange réactionnel obtenu ci-dessus.

Les résultats sont fournis dans le tableau VII. On constate que la transformation de l'oxyde d'éthylène est complète, c'est-à-dire que le taux de conversion de l'oxyde d'éthylène est égal à 1,00. La sélectivité S de la réaction en présence du triflate de zinc, égale à 7,1, est très supérieure à celle obtenue avec l'acétate de potassium (voir exemple 2 comparatif).

*Exemple 31:*

Dans cet exemple, on opère exactement comme à l'exemple 30, excepté le fait qu'au lieu de fixer à 200°C la température de l'enceinte dans laquelle est placé le réacteur tubulaire, on la fixe à 150°C.

Les résultats sont fournis dans le tableau VII. Ils montrent que le taux de conversion de l'oxyde d'éthylène est toujours égal à 1,00. De son côté, la sélectivité S de la réaction, égale à 6,6, reste élevée.

*Tableau VII*

| Exemple | Catalyseur | | Tempé-rature (°C) | Taux de conversion de l'oxyde d'éthylène | S (sélecti-vité) |
|---------|------------|--|-------------------|------------------------------------------|------------------|
| | Nature | Concen-tration (ppm) | | | |
| 30 | $Zn(CF_3SO_3)_2$ | 50 | 200 | 1,00 | 7,1 |
| 31 | $Zn(CF_3SO_3)_2$ | 50 | 150 | 1,00 | 6,6 |

## Revendications

1. Procédé de préparation de produits d'addition d'époxydes et de composés hydroxylés, caractérisé en ce qu'il comporte la réaction, en phase liquide homogène, à une température comprise entre 40 et 250°C, d'une part, d'un époxyde choisi parmi les oxydes d'alcoylène et l'épichlorhydrine et, d'autre part, d'un composé hydroxylé choisi parmi les alcools, les phénols et l'eau, ladite réaction étant effectuée en présence d'un catalyseur constitué par un sel de l'acide trifluorométhane sulfonique.

2. Procédé selon la revendication 1, caractérisé en ce que le sel de l'acide trifluorométhane sulfonique est le trifluorométhane sulfonate d'aluminium.

3. Procédé selon la revendication 1, caractérisé en ce que le sel de l'acide trifluorométhane sulfonique est le trifluorométhane sulfonate de zinc.

4. Procédé selon la revendication 1, caractérisé en ce que l'époxyde est l'oxyde d'éthylène, l'oxyde de propylène ou l'oxyde de butylène.

5. Procédé selon la revendication 1, caractérisé en ce que le composé hydroxylé est un alcool aliphatique comportant de 1 à 20 atomes de carbone ou un monoalcoyléther d'alcoyléneglycol.

6. Procédé selon la revendication 1, caractérisé en ce que la réaction est effectuée à une température comprise entre 80 et 250°C, et de préférence comprise entre 80 et 150°C.

## Patentansprüche

1. Verfahren zur Herstellung von Additionsprodukten von Epoxyden und Hydroxyverbindungen, dadurch gekennzeichnet, dass es die Umsetzung in homogener flüssiger Phase, bei einer Temperatur zwischen 40 und 250°C, einerseits eines Epoxyds, ausgewählt aus Alkylenoxyden und Epichlorhydrin und andererseits einer Hydroxyverbindung, ausgewählt aus Alkoholen, Phenolen und Wasser, umfasst, wobei diese Reaktion in Gegenwart eines Katalysators, bestehend aus einem Salz der Trifluormethansulfonsäure, durchgeführt wird.

2. Verfahren nach Anspruch 1, dadurch gekennzeichnet, dass das Salz der Trifluormethansulfonsäure Aluminiumtrifluormethansulfonat ist.

3. Verfahren nach Anspruch 1, dadurch gekennzeichnet, dass das Salz der Trifluormethansulfonsäure Zinktrifluormethansulfonat ist.

4. Verfahren nach Anspruch 1, dadurch gekennzeichnet, dass das Epoxyd Ethylenoxyd, Propylenoxyd oder Butylenoxyd ist.

5. Verfahren nach Anspruch 1, dadurch gekennzeichnet, dass die Hydroxyverbindung ein aliphatischer Alkohol mit 1-20 Kohlenstoffatomen oder ein Monoalkylether eines Alkylenglykols ist.

6. Verfahren nach Anspruch 1, dadurch gekennzeichnet, dass die Umsetzung bei einer Temperatur zwischen 80 und 250°C und vorzugsweise zwischen 80 und 150°C durchgeführt wird.

## Claims

1. Process for the preparation of addition products of epoxides and hydroxylated compounts, characterised in that it comprises the reaction in a homogeneous liquid phase at a temperature comprised between 40 and 250°C, on the one hand, of an epoxide chosen from amongst alkylene oxides and epichlorhydrin, and on the other hand a hydroxylated compound chosen from amongst alcohols, phenols and water, the said reaction being performed in the presence of a catalyst consisting of a salt of trifluoromethanesulphonic acid.

2. Process according to Claim 1, characterised in that the salt of trifluoromethanesulphonic acid is the trifluoromethanesulphonate of aluminium.

3. Process according to Claim 1, characterised in that the salt of trifluoromethanesulphonic acid is the trifluoromethanesulphonate of zinc.

4. Process according to Claim 1, characterised in that the epoxide is ethylene oxide, propylene oxide or butylene oxide.

5. Process according to Claim 1, characterised in that the hydroxylated compound is an aliphatic alcohol comprising from 1 to 20 carbon atoms or a monoalkyl ether of alkyleneglycol.

6. Process according to Claim 1, characterised in that the reaction is performed at a temperature comprised between 80 and 250°C and preferably comprised between 80 and 150°C.